(19) **Europäisches Patentamt**
**European Patent Office**
**Office européen des brevets**

(11) **EP 1 629 889 B1**

(12) **EUROPEAN PATENT SPECIFICATION**

(45) Date of publication and mention
of the grant of the patent:
**02.05.2012 Bulletin 2012/18**

(51) Int Cl.:
*B01J 23/72* [(2006.01)]     *B01J 27/14* [(2006.01)]
*B01J 27/199* [(2006.01)]     *B01J 37/02* [(2006.01)]
*B01J 37/12* [(2006.01)]     *C07C 51/16* [(2006.01)]

(21) Application number: **04745368.3**

(22) Date of filing: **27.05.2004**

(86) International application number:
**PCT/JP2004/007262**

(87) International publication number:
**WO 2004/105941 (09.12.2004 Gazette 2004/50)**

(54) **PROCESS FOR PRODUCING CATALYST FOR METHACRYLIC ACID PRODUCTION**

VERFAHREN ZUR HERSTELLUNG EINES KATALYSATORS FÜR DIE HERSTELLUNG VON METHACRYLSÄURE

PROCEDE DE PRODUCTION D'UN CATALYSEUR POUR LA PRODUCTION D'ACIDE METHACRYLIQUE

(84) Designated Contracting States:
**CZ DE FR GB**

(30) Priority: **30.05.2003 JP 2003154000**

(43) Date of publication of application:
**01.03.2006 Bulletin 2006/09**

(73) Proprietor: **NIPPON KAYAKU KABUSHIKI KAISHA
Tokyo 102-8172 (JP)**

(72) Inventors:
• **SUDO, Atsushi
Annaka-shi, Gunma 3790133 (JP)**
• **SEO, Yoshimasa
Takasaki-shi, Gunma 3701207 (JP)**
• **KURAKAMI, Tatsuhiko
Ube-shi, Yamaguchi 7590207 (JP)**

(74) Representative: **Gille Hrabal
Patentanwälte
Brucknerstrasse 20
40593 Düsseldorf (DE)**

(56) References cited:
JP-A- 5 031 368     JP-A- 11 043 314
JP-A- 11 226 411     JP-A- 54 161 594
JP-A- 2002 233 760     JP-A- 2002 233 760

**Description**

TECHNICAL FIELD

The present invention relates to a method for preparing catalyst for producing methacrylic acid by subjecting methacrolein, isobutyl aldehyde or isobutyric acid to gas phase catalytic oxidation, having a long -life, a high activity as well as high selectivity.

BACKGROUND ART

A large number of catalysts for producing methacrylic acid through a gas phase catalytic oxidation methacrolein, isobutylaldehyde or isobutyric acid, have been proposed. Most of the catalysts thereof are mainly composed of molybdenum and phosphorus, and have structures of heteropolyacids and/or salts thereof. The catalysts used in the reaction, however, have low reaction activities, low selectivity for the desired substance, and short lifetime, as compared to molybdenum-vanadium based catalysts proposed for producing acrylic acid through a gas phase catalytic oxidation of acrolein, which are known as reactions similar to a gas phase catalytic oxidation of methacrolein, isobutylaldehyde or isobutyric acid. Accordingly, the improvement of catalytic performance of the catalysts is required although some of the catalysts are industrially utilized.

The present inventors first tried the improvement of low activities, low selectivity and short lifetime of conventional gas phase catalytic oxidation catalysts for methacrolein, and found out that gas phase catalytic oxidation catalysts for methacrolein prepared by the addition of a variety of elements to Mo, V and P, have heteropolyacid (salt) structures and have high activities, high selectivity and are particularly stable in the lifetime. The inventors propose the catalysts described in Japanese Patent Publication No. 58-11416, Japanese Patent Publication No. 59-24140, Japanese Patent Publication No. 62-14535 and Japanese Patent Publication No. 62-30177.

Recently, because of high concentrations of raw material gases and of environments under which oxidation reactions are conducted at elevated temperature, catalysts that exhibit further high activities, high selectivity and long lifetime, are needed. Various preparation methods are proposed to provide catalysts that satisfy these demands. For example, Japanese Patent Laid-Open No. 5-31368 for its US-A-5198579 equivalent, and Japanese Patent Laid-Open No. 8-196908 propose methods for preparing molding catalysts that involve using $NH_4$ in addition to the components of Mo, V and P, and utilizing aqueous ammonia as the ammonium source. In addition, Japanese Patent Laid-Open No. 11-226411 describes a method for preparing a molding catalyst that comprises using purified starch when an active component of the catalyst is granulated, and improving the pore volume of the catalyst by burning the starch in the calcining step.

Furthermore, when a catalyst is loaded in a fixed-bed reactor as an industrial catalyst, the catalyst is required to be molded to a constant size in order to reduce the pressure drop of the reaction gas prior to and subsequent to the catalyst layer. For this purpose, known methods involve normally molding a catalyst powder to a cylindrical material, a pellet, a ring-shaped material, a sphere-shaped material, or the like, and impregnating or coating an inert carrier with an active catalyst material also.

Advantages of a coated catalyst having the inert carrier as the core include (i) being capable of improving the effective utilization factor of active components of the catalyst, (ii) being expected to improve the selectivity due to the homogeneous distribution of the residence time of reaction materials within the catalyst, and (iii) facilitating the removal of the reaction heat on account of the improvement of the catalyst thermal conductivity or the dilution effect of the inert carrier. As a result, there are many examples applied to selective oxidation of a large heat release.

On the other hand, technical disadvantages in preparing a coated catalyst include (i) the peeling of the coating layer and the difficulty of obtaining a mechanically strong catalyst because the catalyst is subject to cracking, (ii) the difficulty of coating a carrier with a large amount of active catalytic material, and (iii) the difficulty of obtaining a highly active catalyst due to inclusion of inert materials.

Methods for overcoming the disadvantages are related to the properties of active catalyst substances and the present situation is to study catalysts individually because of no general techniques.

DISCLOSURE OF THE INVENTION

The object of the present invention is to provide a method for preparing a catalyst for producing methacrylic acid in high yield and highly selectively by subjecting methacrolein, isobutyl aldehyde or isobutyric acid to gas phase catalytic oxidation.

The present inventors have tried to improve the low activities, low selectivity and short lifetime of conventional gas phase catalytic oxidation catalysts for methacrolein as a method for solving the above-mentioned problems, and found out that when preparing a catalyst containing the essential components of Mo, V, P, Cu, Cs and $NH_4$, an industrial

catalyst offering a high activity, high selectivity and particularly high stability in the lifetime can be obtained in the case of a particular filtration step being taken. On this base, the present invention came to completion.

That is to say, the present invention relates to:

(3) a method for preparing a coated catalyst for producing methacrylic acid by subjecting methacrolein, isobutyl aldehyde or isobutyric acid to gas phase catalytic oxidation, the method comprising:

(a) a step of blending compounds each containing any one of Mo, V, P, Cu, CS or $NH_4$ and, as needed, a compound containing a metal element other than the above with water to prepare an aqueous solution or dispersion of the compounds (hereinafter referred to, both included, as a slurry);
(b) a step of drying the slurry obtained in the step (a) to obtain a dried slurry;
(c) a step of calcining the dried slurry obtained in the step (b) to obtain a calcined body;
(d) a step of filtering a mixture obtained by blending the calcined body obtained in the step (c) with water to separate an aqueous solution and water-insoluble matter;
(e) a step of drying the water-insoluble matter obtained in the step (d) to obtain a dried water-insoluble body; and
(f) a step of coating a carrier with the dried water-insoluble body obtained in the step (e) using a binder to obtain a coated molded product, as explicitly disclosed in the independent claim 1.

Further features of the claimed invention are disclosed in dependent claims 2-8.

(4) à method for preparing a coated catalyst for producing methacrylic acid by subjecting methacrolein, isobutyl aldehyde or isobutyric acid to gas phase catalytic oxidation, the method comprising:

(a) a step of blending compounds each containing any one of Mo, V, P, Cs or $NH_4$ and, as needed, a compound containing a metal element other than the above, provided that Cu is excluded, with water to prepare an aqueous solution or dispersion of the compounds (hereinafter referred to, both included, as a slurry) ;
(b') a step of drying the slurry obtained in the step (a) to obtain a dried slurry, followed by blending therewith a compound containing Cu, as needed, in the presence of a solvent, further followed, as needed, by drying the obtained mixture, and thereby obtaining a dried body;
(c) a step of calcining the dried body obtained in the step (b') to obtain a calcined body;
(d) a step of filtering a mixture obtained by blending the calcined body obtained in the step (c) with water to separate an aqueous solution and water-insoluble matter;
(e) a step of drying the water-insoluble matter obtained in the step (d) to obtain a dried water-insoluble body; and
(f) a step of coating a carrier with the dried water-insoluble body obtained in the step (e) using a binder to obtain a coated molded product,

(5) the method according to (3) or (4), comprising: the step (a) to (f) and

(g) a step of calcining the coated molded product obtained in the step (f) under an inert gas atmosphere, under an air atmosphere or in the presence of a reducing agent,

(6) the method according to (5), wherein the step (g) is a step of calcining the coated molded product obtained in the step (f) under an inert gas atmosphere,
(7) the method according to any one of (1) to (6), wherein the compound containing Cu is cupper acetate or cupper oxide,
(8) the method according to any one of (1) to (7), wherein the compound containing Cs is a weak acid salt of cesium or cesium hydroxide, a compound containing $NH_4$ is ammonium acetate or ammonium hydroxide,
(9) the method according to any one of (3) to (8), wherein, the compound used as an optional component in the step (a) is a compound containing one or more kinds of elements selected from the group consisting of Sb, As, Ag, Mg, Zn, Al, B, Ge, Sn, Pb, Ti, Zr, Cr, Re, Bi, W, Fe, Co, Ni, Ce, Th, K and Rb,
(10) the method according to any one of (1) to (9), wherein the slurry does not contain an arsenic compound, and
(11) the method according to any one of (3) to (10), wherein the binder is a binder containing ethanol.

BEST MODE FOR CARRYING OUT THE INVENTION

A preparation method according to the invention includes steps of drying and calcining an aqueous solution containing compounds including active ingredients of a catalyst or a water dispersion thereof (hereinafter referred to, both included, as a slurry), followed by blending an obtained calcined body with water, further followed by filtering, and drying a filtration residue.

A preparation method according to the invention includes dissolving and/or dispersing a plurality of compounds each containing one or a plurality of Mo, V, P, Cu, Cs and $NH_4$, and if necessary, elements other than the above (hereinafter, in some cases, these compounds containing these active components are referred to as "active component-containing compound") into water to prepare a slurry (step (a)), drying (step (b)) and calcining (step (c)) the slurry, blending an obtained calcined body with water and filtering the same (step (d)), drying a filtration residue (step (e)), and step (f) as explicitly cited in claim 1.

In the invention, an active component-containing compound used for preparing a slurry is preferably a compound that forms a heteropoly acid or a salt thereof owing to a drying (step (b)) or calcining (step (c)). As these compounds, chlorides, sulfates, nitrates, oxides, or acetates of the active component elements can be included. More specific gexamples of preferable compounds include nitrates such as potassium nitrate and cobalt nitrate; oxides such as molybdenum oxide, vanadium pentoxide, antimony trioxide, cerium oxide, zinc oxide or germanium oxide; and acids such as orthoposphoric acid, phosphoric acid, boric acid, aluminum phosphate or 12-tungstphosphoric acid (or salt thereof). Furthermore, when, as the copper compound, copper acetate (cuprous acetate, cupric acetate, basic copper acetate or cupric oxide, preferably cupric acetate) or copper oxide (cuprous oxide, cupric oxide) is used, in some cases, a favorable effect can be obtained. Still furthermore, as the cesium compound, cesium acetate or cesium hydroxide and cesium weak acid salt, and, as the ammonium compound, both of ammonium acetate and ammonium hydroxide are preferably used. The compounds including the active components can be used singularly or in combinations of two or more kinds thereof. As the cesium weak acid salt, as far as it is a salt of cesium and a generally known weak acid, there is no particular restriction on. For instance, cesium hydrogen carbonate, cesium carbonate, and cesium acetate can be included, and among them, cesium acetate is preferable. Among them, as to cesium acetate, commercially available one can be used as it is; however, by adding acetic acid more than an equivalent weight to an aqueous solution of water-soluble salts such as cesium hydroxide and cesium carbonate, it can be added as an aqueous solution of cesium acetate. Alternatively, an aqueous solution of cesium hydroxide can be added as it is.

In the invention, as the active components other than Mo, V, P, Cu, Cs and $NH_4$, one or more kinds selected from the group consisting of As, Sb, Ag, Mg, Zn, Al, B, Ge, Sn, Pb, Ti, Zr, Cr, Re, Bi, W, Fe, Co, Ni, Ce, Th, K and Rb can be included. Among thm, the elements other than As are preferable.

In the invention, ratios of active component-containing compounds used are, in atomic ratios thereof with respect to 10 of molybdenum, normally 0.1 or more and 6 or less for vanadium, preferably 0.3 or more and 2.0 or less; normally 0.5 or more and 6 or less for phosphorus, preferably 0.7 or more and 2.0 or less; normally more than 0 and 3 or less for copper, preferably 0.01 or more and 1 or less; normally 0.01 or more and 4.0 or less for cesium, preferably 0.1 or more and 2.0 or less; and normally 0.1 or more and 4.0 or less for ammonium, preferably 0.5 or more and 3.0 or less. The kinds and the ratios of other active components that are used as needed are appropriately determined so that in conformity with conditions used a catalyst that shows the optimum performance may be obtained.

The preparation method according to the invention is performed according to a process mentioned below.

Firstly, a slurry of active component-containing compounds is prepared (step (a)). The slurry can be obtained by uniformly blending the respective active component-containing compounds and water. The slurry preferably contains all of the active component-containing compounds in their necessary amounts as a catalyst. There is no particular restriction on an order of addition of the compounds containing active components when the slurry is prepared. However, it is preferable to firstly put compounds containing Mo, V, P and other metal elements, if necessary, into a slurry, followed by adding a cesium-containing compound, an ammonium-containing compound and a copper-containing compound in the slurry.

There is no particular restriction on a temperature when the slurry is prepared as far as it is in the range that does not disturb the preparation. However, when a temperature when the cesium-containing compound, the ammonium-containing compound and the copper-containing compound are added is normally in the range of 0 to 35°C and preferably in the range of 10 to 30°C, in some cases, an obtained catalyst may have higher activity. As this tendency becomes more obvious when copper acetate is used as the copper compound, a method for preparing a slurry becomes more efficient when the preferable adding method is adopted.

In the invention, it is preferable that the slurry is an aqueous solution. There is no particular restriction on an amount of water used in the slurry insofar as it is an amount that can completely dissolve a total amount of compounds used or can uniformly mix them. However, it is appropriately determined in consideration of a drying method and a drying condition mentioned below. Usually, an amount of water used is substantially 200 to 2000 parts by mass relative to 100 parts by mass of a total mass of the compounds for slurry preparation. An amount of water may be more than necessary, however, when it is excessive, disadvantages such as that energy cost during the drying step becomes higher and in some cases incomplete drying may be caused are generated. That is, since advantage is less in comparison with disadvantage, an appropriate amount is preferable.

Next, the slurry obtained in the above is dried, and thereby a dried slurry is obtained (step (b)). There is no particular restriction on a drying method insofar as the slurry is completely dried. Examples of the drying method include a drum drying, a freeze dry, a spray drying and evaporation to dryness. Among these methods, in the invention, it is preferable to use the spray drying that can dry from a slurry state to powder or granulated powder in short time, or the evaporation to dryness that can directly dry the slurry and is simple, the evaporation to dryness being particularly preferable.

A drying temperature of the spray drying differs depending on a concentration of the slurry and the speed of supplying the slurry. However, generally, the temperature at the exit of the drying machine is within the range of 70 to 150°C. Furthermore, it is preferable to dry so that an average particle diameter of an obtained dried slurry may be in the range of 30 to 700 $\mu$m. In the case of the evaporation to dryness, in particular, the dried slurry can be obtained in the shape of a lump or a large particle. Accordingly, it is preferable to pulverize appropriately, preferably so as to be 700 $\mu$m or less to use. Thus, according to the invention, pulverized one is also contained in the dried slurry.

Then, the dried slurry obtained in this way is calcined preferably in an air atmosphere and thereby a calcined body is obtained (step (c)). In this case it is ordinary calcined at a temperature in the range of 100 to 420°C, and preferably in the range of 250 to 400°C. The preferable calcining time is 1 to 20 hr. Such calcined body is preferably obtained as powder.

Next the calcined body is blended with appropriate amount of water, preferably 2 to 5 times by mass as the calcined body, and thereby water-soluble components are eluted. The water-soluble components are assumed to be compounds having the heteropoly acid structure. Since the heteropoly acid salts aimed in the invention exists without dissolving into water, it is separated by filtering (step (d)), dried (step (e)), and coating according to step (f) in the wording of claim 1. Thus obtained dried body is preferably powder, and can be used as it is as a catalyst for the gas phase catalytic oxidation.

According to the invention includes blending a plurality of compounds each containing one or a plurality of Mo, V, P, Cs and NH$_4$, and if necessary, elements other than the above, provided that Cu is excluded, with water to prepare a slurry (step (a)), drying the same, blending an obtained dried slurry with a Cu-containing compound in the presence of a solvent if necessary, drying it if necessary to obtain a dried body (step (b')), followed by calcining (step (c)), blending an obtained calcined body with water and filtering the same (step (d)), drying a filtration residue (step (e)), and coating (step f) and using it as a catalyst active component. Thus obtained dried body can be used as it is as a catalyst for the gas phase catalytic oxidation.

According to the invention, a step of obtaining a catalyst active component, except that a copper-containing compound is blended separately from a dried slurry of other active components-containing compounds, can be carried out in kinds of the active component-containing compounds and ratios used and other conditions. The dried slurry and the Cu-containing compound can be blended in powder, alternatively the Cu-containing compound may be blended with a solvent, preferably with water to form a slurry. In this case, an amount of water used may be comparable as that used in the first embodiment. A mixture of the dried slurry and the Cu-containing compound, in the case of water being used during preparation, is appropriately dried to obtain a dried body. Then, the dried body is calcined similarly to the first embodiment, followed by blending with water and drying a filtration residue, and thereby a target catalyst can be obtained. The later-added copper compound, after the step of drying (and calcining), does not exist as a free compound.

Thus obtained dried water-insoluble body, that is, the catalyst active component, in order to make a pressure loss of a reaction gas smaller, is preferably used after forming into a cylindrical matter, a pellet, a ring, or a sphere. Among them, as an improvement in the selectivity and the elimination of reaction heat can be expected, it is most preferable to coat an inactive carrier with an dried water-insoluble body to form a molded body coated with the catalyst.

In the coating step (step (f)), a rolling granulation method described later can be preferably applied. According to the method, in apparatus having a flat or irregular disc at, for instance, a bottom portion of a fixed vessel, the disc is rotated at a high-speed, and thereby carriers in the vessel are violently agitated by repeating autorotation and revolution. When a binder and the dried water-insoluble body as well as, if necessary, other additives such as a mixture of a molding aid and a strength improver are added in the vessel in rotation, the mixture is coated on the carriers. As a method of adding a binder, (i) a method of blending the binder with the mixture beforehand, (ii) a method of adding the binder into the fixed vessel simultaneously with the mixture, (iii) a method of adding the binder after the mixture is added into the fixed vessel, (iv) a method of adding the binder before the mixture is added into the fixed vessel, and (v) a method of dividing the mixture and the binder separately and by appropriately combining the methods (ii) to (iv) to add a total amount can be arbitrarily adopted. Among these, in the case of method (v), it is preferable to control an addition speed by use of an auto feeder and so on so that a predetermined amount may be carried on carriers without, for instance, sticking of the mixture to a wall of the fixed vessel and coagulation between the mixtures.

There is no particular restriction on the binder insofar as it is at least one kind selected from the group consisting of water and organic compounds having a boiling point of 150°C or less at one atmosphere. However, when the drying after the coating is taken into consideration, ones having a boiling point of 100°C or less are preferable. Specific examples of the binders other than water include alcohols such as methanol, ethanol, propanols, and butanols, preferably alcohols having 1 to 4 carbon atoms; ethers such as ethyl ether, butyl ether or dioxane; esters such as ethyl acetate or butyl acetate; ketones such as acetone or methyl ethyl ketone; and aqueous solutions thereof. Among them, ethanol is particularly preferable. In the case of ethanol being used as a binder, ethanol/water is in the range of 10/0 to 1/9 (by mass ratio) and preferably in the range of 10/0 to 7/3 (by mass ratio). When a mixture of ethanol and water is used, the limit of inflammability thereof should be considered depending on the drying conditions. In such a case, ethanol concentration is preferably in the range of 15 to 40% by mass. An amount of these binder used is normally in the range of 2 to 60 parts by mass relative to 100 parts by mass of the dried water-insoluble body, and preferably in the range of 5 to 25 parts by mass.

Specific examples of the carrier used in the invention include spherical carriers made of silicon carbide, alumina, silica alumina, mullite and alundum and having a diameter in the range of 1 to 15 mm, and preferably in the range of 2.5 to 10 mm. As the carrier, in ordinary cases, one having a pore content of 10 to 70% is used. A ratio of the carrier to the dried water-insoluble body used for coating is normally in the range of 10 to 75% by mass in terms of dried water-insoluble body /(dried water-insoluble body + carrier), and preferably in the range of 15 to 60% by mass.

In the case where a ratio of the dried water-insoluble body used for coating is large, the reactivity of the coated catalyst becomes larger on the one hand, on the other hand, the mechanical strength tends to become smaller (wearing ratio tends to become larger). On the contrary, when the ratio of the dried water-insoluble body being coated is small, the mechanical strength becomes larger (wearing ratio becomes smaller) on the one hand, on the other hand, the reactivity tends to become smaller.

In the invention, in the case of the carrier being coated with the dried water-insoluble body, as needed, a molding aid such as silica gel, diatom earth and alumina powder can be added to the dried water-insoluble body. An amount of the molding aid added is ordinarily 5 to 60 parts by mass relative to 100 parts by mass of the dried water-insoluble body.

Furthermore, it is useful in improving the mechanical strength of the catalyst to add to the catalyst component inorganic fiber such as ceramics fiber and whisker inactive to the catalyst component as the strength improver. However, such fibers as potassium titanate whisker and basic magnesium carbonate whisker that can react with the catalyst component are not suitable. An amount of these fibers added is ordinarily in the range of 1 to 30 parts by mass relative to 100 parts by mass of the dried water-insoluble body.

The additive agents such as the molding aid and the strength improver are added, ordinary during the coating step, in the granulator along with the carrier, the dried water-insoluble body and the binder and used for coating the carrier.

In this way, the carrier is coated with the dried water-insoluble body, and a diameter of the coated material obtained by this process is ordinarily in the range of about 3 to 15 mm.

The coated catalyst obtained in this way can be used in the gas phase catalytic oxidation as a catalyst without modification. However, it is preferably calcined (step (g)) because in some cases the catalytic activity is improved by calcining. In this case, a calcining temperature is ordinarily in the range of 100 to 450°C, and preferably 250 to 420°C. A calcining time is preferably in the range of 1 to 20 hr.

In addition, the calcining is ordinarily carried out under an air atmosphere. However, it may be carried out under an inactive gas atmosphere such as nitrogen, and after the calcining under the inactive gas atmosphere, calcining under an air atmosphere can be further applied as circumstances demand. Furthermore, it is preferable to calcine under the inactive gas atmosphere, more preferably in the presence of a reducing agent, as more active catalyst can be obtained in some cases. There is no particular restriction on the reducing agent insofar as it preferably becomes a gas at a calcining temperature. Examples of the reducing agent include CO, alcohols, aldehydes, ketones and organic acids having 2 to 5 carbon atoms, and ethanol is particularly preferable.

The catalyst obtained in the way as mentioned above (hereinafter referred to as catalyst according to the invention) is used for preparing methacrylic acid through the gas phase catalytic oxidation of methacrolein, isobutyl aldehyde or isobutyric acid. When the phrase "the catalyst according to the invention" is used without specific notice, it is used to denote both of the dried water-insoluble body obtained through the steps (a) to (e) and the coated catalyst obtained further through the step (f) (preferably, also step (g)).

The gas phase catalytic oxidation that uses methacrolein, which is amost suitable rawmaterial to use the catalyst according to the invention, will be explained below.

In the gas phase catalytic oxidation, molecular oxygen or molecular oxygen-containing gas is used. A ratio of molecular oxygen used to methacrolein is in the range of 0. 5 to 20 by mole ratio relative, and particularly preferably in the range of 1 to 10. To make the reaction process proceed smoothly, it is preferable to add water to a raw material gas in the range of 1 to 20 by mole ratio relative to methacrolein.

The raw material gas may contain, other than oxygen and asneededwater (in ordinary cases it is contained as water vapor), gases inert to the reaction such as nitrogen, carbon dioxide gas, and saturated hydrocarbons.

Furthermore, as for methacrolein, gases obtained by oxidizing isobutylene, tert-buthanol and methyl-tert-butyl ether can be supplied without modification.

A reaction temperature during the gas phase catalytic oxidation is ordinarily in the range of 200 to 400°C, and preferably in the range of 260 to 360°C. A supply amount of the raw material gas is, in terms of spatial velocity (SV), ordinarily in the range of 100 to 6000 hr$^{-1}$ and preferably in the range of 400 to 3000 hr$^{-1}$.

In the case of the catalyst according to the invention being used, there is no great change in a reaction result even when the SV is increased. Accordingly, it is possible to perform the reaction at a high spatial velocity.

Furthermore, though it is possible to perform the gas phase catalytic oxidation either under a pressure or a reduced pressure, in general, a pressure around an atmospheric pressure is suitable.

(Examples)

In what follows, the present invention will be more specifically explained with examples.

A conversion ratio, selectivity and yield in these examples are defined as follows.

- 
  - Conversion ratio = (a number of moles of reacted methacrolein/a number of moles of supplied methacrolein) × 100
- 
  - Selectivity = (a number of moles of generated methacrylic acid/a number of moles of reacted methacrolein) × 100
- 
  - Yield = (a number of generated methacrylic acid/a number of moles of supplied methacrolein) × 100

[Example 1]

1) Preparation of catalyst

In 2100 ml of purified water, 300 g of molybdenum trioxide, 13.26 g of vanadium pentoxide and 27.62 g of 85% by mass orthophosphoricacidwereadded, followedbyheatingunderreflux at a temperature in the range of 90 to 100°C for 5 hr, and thereby a umbered transparent solution was obtained. Then, 12.15 g of antimony trioxide was added thereto, followed by further heating under reflux at a temperature in the range of 90 to 100°C for 2 hr, and thereby a navy blue solution in which antimony trioxide was dissolved was obtained. Subsequently, the solution was cooled to a temperature of 15 to 20°C, followed by gradually adding under agitation 20.00 g of cesium acetate dissolved in 150 ml of purified water and 24.09 g of ammonium acetate dissolved in 150 ml of purifiedwater together, further followedby ripening at a temperature of 15 to 20°C for 1 hr, and thereby an aeruginous slurry containing a cesium salt of a heteropoly acid precursor and an ammonium salt was obtained.

Then, 16.64 g of cupric acetate monohydrate dissolved in 240 ml of purified water was added to the slurry, further followed by ripening at a temperature of 15 to 20°C for 15 min.

Next, the slurry was dried by evaporation to dryness by use of hot water, followed by crushing with a mortar, further followed by classifying to 700 $\mu$m or less, still further followed by subjecting primary calcining at 310°C under air flow for 5 hr, and thereby calcined granulated powder was obtained. A composition of the calcined granulated powder was, by charge ratio,

$$Mo_{10}V_{0.7}P_{1.15}Cu_{0.4}Sb_{0.4}Cs_{0.5} (NH_4)_{1.5}.$$

Subsequently, 300g of the obtained calcined granulated powder was dispersed in 1000 ml of purified water, followed by agitating at 40°C for 1 hr. Then the dispersion liquid was filtered, and filtered greenish white water-insoluble matter (filtration residue) and a umber filtrate were separately evaporated to dryness with hot water, followed by pulverizing to 300 to 700 $\mu$m, further followed by classifying, and thereby granular catalyst (A) was obtained from the filtration residue, and solidified matter for comparison (A) was obtained from the filtrate.

In this case, a separation ratio between the filtration residue and the filtrate was 86.8% by mass of the filtration residue and 13.2% by mass of the filtrate. It was found that an atomic ratio of metal elements of the obtained granular catalyst (A) was

$$Mo_{10}V_{0.5}P_{1.8}.CU_{0.32}Sb_{0.41}CS_{0.46}$$

by fluorescent X-ray analysis, the granular catalyst was a salt of heteropoly acid by X-ray diffraction analysis, and nitrogen (i.e. $NH_4$; and so forth) was contained by CHN analysis.

Furthermore, it was found that an atomic ratio of metal elements of the obtained solidified matter for comparison (A) was

$$Mo_{10}V_{1.53}P_{2.46}Cu_{1.14}Sb_{0.13}$$

by fluorescent X-ray analysis, the solidified matter was a precursor that did not have heteropoly acid by X-ray diffraction analysis, and nitrogen was not contained by CHN analysis.

Then, 176.8 g of one that was obtained by pulverizing and classifying the obtained granular catalyst (A) to 300 $\mu$m or less and 25.9 g of a strength improver (ceramic fiber) were blended uniformly, followedby coating and molding on 173.7 g of spherical porous alumina carrier (particle diameter 3.5 mm) by use of an aqueous solution of 90% by mass of ethanol as a binder. Then an obtained molded body was subjected to secondary calcining at a temperature of 310°C for 5 hr under air flow, and thereby a coated catalyst aimed at (A) was obtained.

2) Catalytic oxidation of methacrolein

In a stainless steel reactor tube having an inner diameter of 18.4mm, 10.3 ml of the obtained coated catalyst (A) was filled. A catalytic oxidation of methacrolein was conducted under the conditions of a raw material gas composition (in molar ratio) of methacrolein: oxygen: water vapor: nitrogen = 1: 2: 4: 18.6, the space velocity (SV) of 1200 hr$^{-1}$, and a reaction bath temperature of 310°C. The reaction was at first kept at a reaction bath temperature of 310°C for 3 hr, and the reaction bath temperature was raised to 350°C and kept there for 15 hr. Subsequently, the reaction bath temperature was lowered to 310°C and a reaction result was measured. The results are shown in Table 1. Furthermore, 4.0 g of the granulated catalyst (A) and the same amount of the solidified matter for comparison (A) were separately blended with 8.0 g of quartz sand of 300 to 700 $\mu$m and used in the oxidation reaction under the same reaction conditions as the above. The results are shown together in Table 1.

[Table 1]

|  | Peak temperature (°C) | Conversion ratio (%) | Selectivity (%) | Yield (%) |
|---|---|---|---|---|
| Coated catalyst (A) | 325 | 88.5 | 82.7 | 73.1 |
| Granular catalyst (A) | - | 87.6 | 86.7 | 75.9 |
| Solidified matter for comparison (A) | - | 40.6 | 83.1 | 33.7 |

(Comparative Example 1)

1) Preparation of Catalyst

In 2100 ml of purified water, 300 g of molybdenum trioxide, 13.26 g of vanadium pentoxide and 27.62 g of 85% by mass orthophosphoricacidwereadded, followedbyheatingunderreflux at a temperature in the range of 90 to 100°C for 5 hr, and thereby a umbered transparent solution was obtained. Then, 12.15 g of antimony trioxide was added thereto, followed by further heating under reflux at a temperature in the range of 90 to 100°C for 2 hr, and thereby a navy blue solution in which antimony trioxide was dissolved was obtained. Subsequently, the solution was cooled to a temperature of 15 to 20°C, followed by gradually and simultaneously adding under agitation 20.00 g of cesium acetate dissolved in 150 ml of purified water and 24.09 g of ammonium acetate dissolved in 150 ml of purified water, further followed by ripening at a temperature of 15 to 20°C for 1 hr, and thereby a aeruginous slurry containing a cesium salt of a heteropoly acid precursor and an ammonium salt was obtained.

Then, 16.64 g of cupric acetate monohydrate dissolved in 240 ml of purified water was added to the slurry, further followed by ripening for 1 hr.

Next, the slurry was dried by evaporation to dryness by use of hot water, followed by pulverizing, further followed by classifying to 300 to 700 $\mu$m, and thereby solidified matter for comparison (B) was obtained.

A composition of the solidified matter for comparison (B) at this time was, by charge ratio,

$$Mo_{10}V_{0.7}P_{1.15}Cu_{0.4}Sb_{0.4}Cs_{0.5}(NH_4)_{1.5}.$$

Then, the obtained solidified matter for comparison (B) was pulverized and classified to 300 $\mu$m or less, and 365.4 g thereof and 52.1 g of a strength improver (ceramic fiber) were blended uniformly, followed by coating and molding on 349.8 g of spherical porous alumina carrier (particle diameter 3.5 mm) by use of an aqueous solution of 90% by mass of ethanol as a binder. Then an obtained molded body was subjected to calcining at a temperature of 310°C for 5 hr under air flow, and thereby a coated catalyst for comparison (B) was obtained.

2) Catalytic oxidation of methacrolein

The obtained coated catalyst for comparison (B) was used in a catalytic oxidation of methacrolein similarly to example 1, and a reaction result was measured in the same way as example 1. The results are shown in Table 2. Moreover, 4.0 g of the solidified matter for comparison (B) and 8.0 g of the quartz sand of 300 to 700 $\mu$m were blended and used in the oxidation reaction under the same reaction conditions as the above. The results are shown together in Table 2.

[Table 2]

|  | Peak temperature (°C) | Conversion ratio (%) | Selectivity (%) | Yield (%) |
|---|---|---|---|---|
| Coated catalyst (B) | 322 | 82.7 | 83.1 | 68.7 |
| Solidified matter for comparison (B) | - | 74.1 | 89.4 | 66.3 |

[Example 2]

1) Preparation of Catalyst

In 2100 ml of purified water, 300 g of molybdenum trioxide, 13.26 g of vanadium pentoxide and 27.62 g of 85% by mass orthophosphoricacidwereadded, followedbyheatingunderreflux at a temperature in the range of 90 to 100°C for 5 hr, and thereby a umbered transparent solution was obtained. Then, 12.15 g of antimony trioxide was added thereto, followed by further heating under reflux at a temperature in the range of 90 to 100°C for 2 hr, and thereby a navy blue solution in which antimony trioxide was dissolved was obtained. Subsequently, the solution was cooled to a temperature of 15 to 20°C, followed by gradually and simultaneously adding under agitation 20.00 g of cesium acetate dissolved in 150 ml of purified water and 24.09 g of ammonium acetate dissolved in 150 ml of purified water, further followed by ripening at a temperature of 15 to 20°C for 1 hr, and thereby a aeruginous slurry containing a cesium salt of a heteropoly acid precursor and an ammonium salt was obtained.

Next, the slurry was dried by evaporation to dryness by use of hot water, followed by pulverizing with a mortar, further followed by classifying to 700 $\mu$m or less, and thereby pulverized body was obtained. A composition of the pulverized body at this time was, by charge ratio,

$$Mo_{10}V_{0.7}P_{1.15}Cu_{0.4}Sb_{0.4}Cs_{0.5}(NH_4)_{1.5}.$$

Then, to the pulverized body, 16.64 g of cupric acetate monohydrate was added in powder, followed by adding 100 g of an aqueous solution of 90% by mass of ethanol, further followed by kneading, still further followed by once more evaporating to dryness with hot water, followed by pulverizing with a mortar to 700 $\mu$m or less, further followed by subjecting to a primary calcining under air flow at 310°C for 5 hr, and thereby calcined granule (C) was obtained. A composition of the granulated body at this time was, by charge ratio,

$$Mo_{10}V_{0.7}P_{1.15}Cu_{0.4}Sb_{0.4}Cs_{0.5}(NH_4)_{1.5}.$$

Furthermore, it was assumed from by X-ray diffraction analysis that added copper was not isolated as copper acetate or copper oxide.

Subsequently, 356 g of the obtained calcined granule (C) was dispersed in 1317 ml of purified water, followed by agitating at 40°C for 1 hr. Then the dispersion liquid was filtered, and filtered greenish white water-insoluble matter (filtration residue) and a umber filtrate were separately evaporated to dryness with hot water, followed by pulverizing to 300 to 700 $\mu$m, further followed by classifying, and thereby granular catalyst (C) was obtained from the filtration residue, and solidified matter for comparison (C) was obtained from the filtrate.

At this time, a separation ratio between the filtration residue and the filtrate was 88.4% by mass of the filtration residue and 11.6% by mass of the filtrate.

Then, 324.4 g of one that was obtained by pulverizing and classifying the obtained granular catalyst (C) to 300 $\mu$m or less and 47.4 g of a strength improver (ceramic fiber) were blended uniformly, followedbycoatingandmoldingon318.2gofspherical porous alumina carrier (particle diameter 3.5 mm) by use of an aqueous solution of 90% by mass of ethanol as a binder. Then the obtained molded body was subjected to secondary calcining under air flow at a temperature of 310°C for 5 hr, and thereby a coated catalyst aimed at (C) was obtained.

2) Catalytic oxidation of methacrolein

The obtained coated catalyst (C) was used in a catalytic oxidation of methacrolein similarly to example 1, and a reaction result was measured in the same way as example 1. The results are shown in Table 3. Moreover, 4.0 g of the calcined granule (C), the granular catalyst (C) and the solidified matter for comparison (C) each and 8.0 g of the quartz sand of 300 to 700 $\mu$m were blended and used in the oxidation reaction under the same reaction conditions as the above. The results are shown together in Table 3.

[Table 3]

|  | Peak temperature (°C) | Conversion ratio (%) | Selectivity (%) | Yield (%) |
|---|---|---|---|---|
| Coated catalyst (C) | 328 | 92.2 | 82.3 | 75.9 |
| Calcined granule (C) | - | 88.2 | 87.5 | 77.2 |
| Granular catalyst (C) | - | 93.7 | 86.4 | 81.0 |
| Solidified matter for comparison (C) | - | 35.8 | 82.8 | 29.6 |

(Comparative example 2)

1) Preparation of catalyst

In 2100 ml of purified water, 300 g of molybdenum trioxide, 13.26 g of vanadium pentoxide and 27.62 g of 85% by mass orthophosphoricacidwereadded, followedbyheatingunderreflux at a temperature in the range of 90 to 100°C for 5 hr, and thereby a umbered transparent solution was obtained. Then, 12.15 g of antimony trioxide was added thereto, followed by further heating under reflux at a temperature in the range of 90 to 100°C for 2 hr, and thereby a navy blue solution in which antimony trioxide was dissolved was obtained. Subsequently, the solution was cooled to a temperature of 15 to 20°C, followed by gradually and simultaneously adding under agitation 20.00 g of cesium acetate dissolved in 150 ml of purified water and 24.09 g of ammonium acetate dissolved in 150 ml of purified water, further followed by ripening at a temperature of 15 to 20°C for 1 hr, and thereby an aeruginous slurry containing a cesium salt of a heteropoly acid precursor and an ammonium salt was obtained.

Subsequently, the slurry was dried by evaporation to dryness by use of hot water, followed by pulverizing by use of a mortar, further followed by classifying to 300 $\mu$m or less, and thereby pulverized matter was obtained. A composition of the pulverized matter was, by charge ratio,

$$Mo_{10}V_{0.7}P_{1.15}Sb_{0.4}Cs_{0.5}(NH_4)_{1.5.}$$

Then, 16. 64 g of cupric acetate monohydrate in powder was added to the pulverized matter, further followed by mixing, and thereby a mixture was obtained.

Then, 381.5 g of the pulverized matter and 53.8 g of a strength improver (ceramic fiber) were blended uniformly, followed by coating and molding on 358.4 g of spherical porous alumina carrier (particle diameter 3.5 mm) by use of an aqueous solution of 90% by mass of ethanol as a binder. Then an obtained molded body was calcined under air flow at a temperature of 310°C for 5 hr, and thereby a coated catalyst for comparison (D) was obtained.

A composition of a catalyst active component after the calcining (the pulverized matter) was, by charge ratio,

$$Mo_{10}V_{0.7}P_{1.15}Cu_{0.4}Sb_{0.4}Cs_{0.5}(NH_4)_{1.5}.$$

Furthermore, it was assumed from X-ray diffraction analysis that added copper was not isolated as copper oxide or copper oxide.

2) Catalytic oxidation of methacrolein

The coated catalyst (D) was used in a catalytic oxidation of methacrolein similarly to example 1, and a reaction result was measured in the same way as example 1. The results are shown in Table 4.

[Table 4]

| | Peak temperature (°C) | Conversion ratio (%) | Selectivity (%) | Yield (%) |
|---|---|---|---|---|
| Coated catalyst (C) | 326 | 88.5 | 82.5 | 73.0 |

[Example 3]

1) Preparation of catalyst

In 2100 ml of purified water, 300 g of molybdenum trioxide, 15.16 g of vanadium pentoxide and 27.62 g of 85% by mass orthophosphoric acid were added, followed by heating under reflux at a temperature in the range of 90 to 100°C for 5 hr, and thereby a umbered transparent solution was obtained. Subsequently, the solution was cooled to a temperature of 15 to 20°C, followed by gradually and simultaneously adding under agitation 17.49 g of cesium hydroxide monohydrate dissolved in 100 ml of purified water and 20.88 g of ammonium acetate dissolved in 100 ml of purified water, further followed by ripening at a temperature of 15 to 20°C for 15 min, and thereby a yellowish-white slurry containing a cesium salt of a heteropoly acid precursor and an ammonium salt was obtained.
Then, 16.64 g of cupric acetate monohydrate dissolved in 200 ml of purified water was added to the slurry, further followed by ripening at a temperature of 15 to 20°C for 1 hr.
In the next place, the slurry was dried by evaporation to dryness by use of hot water, followed by pulverizing with a mortar, further followed by classifying to 700 μm or less, still further followed by subjecting to primary calcining under air flow at 310°C for 5 hr, and thereby calcined granule was obtained. A composition of the calcined granule (E) was, by charge ratio,

$$Mo_{10}V_{0.8}P_{1.15}Cu_{0.4}Sb_{0.4}Cs_{0.5}(NH_4)_{1.3}.$$

Subsequently, 351.5 g of the obtained calcined granule was dispersed in 1300 ml of purified water, followed by agitating at 40°C for 1 hr. Then the dispersion liquid was filtered, and filtered yellowish-white water-insoluble matter (filtration residue) and a umber filtrate were separately evaporated to dryness with hot water, followed by pulverizing to 300 to 700 μm, further followed by classifying, and thereby granular catalyst (E) was obtained from the filtration residue, and solidified matter for comparison (E) was obtained from the filtrate.
In this case, a separation ratio between the filtration residue and the filtrate was 78.9% by mass of the filtration residue and 21.1% by mass of the filtrate.
Subsequently, 245.0 g of one that was obtained by pulverizing and classifying the obtained granular catalyst (E) to 300 μm or less and 36.2 g of a strength improver (ceramic fiber) were blended uniformly, followed by coating and molding on 243.4 g of spherical porous alumina carrier (particle diameter 3.5 mm) by use of an aqueous solution of 90% by mass of ethanol as a binder. Then the obtained molded body was divided equally into two, (i) one was calcined (secondary calcining) in a box type hot air calcining furnace under nitrogen flow (5 L/min) in the presence of ethanol (20 g/hr) as a reducing agent at 380°C for 10 hr, and thereby a targeted coated catalyst (E-1) was obtained, and (ii) the other was subjected to secondary calcining in a box type hot air calcining furnace under air flow at 380°C for 10 hr, and thereby a targeted coated catalyst (E-2) was obtained.

2) Catalytic oxidation of methacrolein

Obtained coated catalysts (E-1) and (E-2) were used in the catalytic oxidation of methacrolein similarly to example 1, and reaction results were measured similarly to example 1. Results thereof are shown in Table 5. Furthermore, 4.0 g of each of the granular catalyst (E) and the solidified matter for comparison (E) was mixed with 8.0 g of quartz sand of 300 to 700 $\mu$m, the mixtures each were used in the oxidation reaction under the same reaction conditions as that of example 1. Results are shown together in Table 5.

[Table 5]

|  | Peak temperature (°C) | Conversion ratio (%) | Selectivity (%) | Yield (%) |
|---|---|---|---|---|
| Coated catalyst (E-1) | 321 | 78.0 | 87.7 | 68,4 |
| Coated catalyst (E-2) | 320 | 76.9 | 87.4 | 67.2 |
| Granular catalyst (E) | - | 78.2 | 89.9 | 70.3 |
| Solidified matter for comparison (E) | - | 46.2 | 84.8 | 39.2 |

(Comparative Example 3)

1) Preparation of catalyst

In 2100 ml of purified water, 300 g of molybdenum trioxide, 15.16 g of vanadium pentoxide and 27.62 g of 85% by mass orthophosphoric acid were added, followed by heating under reflux at a temperature in the range of 90 to 100°C for 5 hr, and thereby a umbered clear solution was obtained. Subsequently, the solution was cooled to a temperature of 15 to 20°C, followed by gradually and simultaneously adding under agitation 17.49 g of cesium hydroxide monohydrate dissolved in 100 ml of purified water and 20.88 g of ammonium acetate dissolved in 100 ml of purified water, further followed by ripening at a temperature of 15 to 20°C for 15 min, and thereby a yellowish-white slurry containing a cesium salt of a heteropoly acid precursor and an ammonium salt was obtained.
Then, 16.64 g of cupric acetate monohydrate dissolved in 200 ml of purified water was further added to the slurry, further followed by ripening for 1 hr.
In the next place, the slurry was dried by evaporation to dryness by use of hot water, followed by pulverizing with a mortar, further followed by classifying to 300 to 700 $\mu$m, and thereby pulverized matter (F) was obtained. A composition of the pulverized matter (F) was, in terms of charge ratio,

$$Mo_{10}V_{0.8}P_{1.15}Cu_{0.4}Sb_{0.4}Cs_{0.5}(NH_4)_{1.3}.$$

Subsequently, the obtained pulverized matter was pulverized to 300 $\mu$m or less and classified, 334.0 g thereof and 48.6 g of a strength improver (ceramic fiber) were blended uniformly, followedby coating and molding on 326. 6 gof spherical porous alumina carrier (particle diameter 3.5 mm) by use of an aqueous solution of 90% by mass of ethanol as a binder. Then the obtained molded body was divided equally into two, (i) one was calcined (secondary calcining) in a box type hot air calcining furnace under nitrogen flow (5 L/min) in the presence of ethanol (20 g/hr) as a reducing agent at 380°C for 10 hr, and thereby a targeted coated catalyst (F-1) was obtained, and (ii) the other was subjected to secondary calcining in a box type hot air calcining furnace under air flow at 380°C for 10 hr, and thereby a targeted coated catalyst (F-2) was obtained.

2) Catalytic oxidation of methacrolein

Obtained coated catalysts (F-1) and (F-2) were used in the catalytic oxidation of methacrolein similarly to example 1, and reaction results were measured similarly to example 1. Results thereof are shown in Table 6. Furthermore, 4.0 g of the pulverized matter (F) was mixed with 8.0 g of quartz sand of 300 to 700 $\mu$m, the mixture was used in the oxidation reaction under the same reaction conditions as that of example 1. Results are shown together in Table 6.

[Table 6]

|  | Peak temperature (°C) | Conversion ratio (%) | Selectivity (%) | Yield (%) |
|---|---|---|---|---|
| Coated catalyst (F-1) | 317 | 53.9 | 85.6 | 46.1 |
| Coated catalyst (F-2) | 318 | 61.2 | 85.9 | 52.6 |

(continued)

|  | Peak temperature (°C) | Conversion ratio (%) | Selectivity (%) | Yield (%) |
|---|---|---|---|---|
| Pulverized matter (F) | - | 69.2 | 88.5 | 61.3 |

[Example 4]

1) Preparation of catalyst

In 2100 ml of purified water, 300 g of molybdenum trioxide, 15.16 g of vanadium pentoxide and 27.62 g of 85% by mass orthophosphoricacidwereadded, followedbyheatingunderreflux at a temperature of 90 to 100°C for 5 hr, and thereby a umber clear solution was obtained. Then, 1.52 g of antimony trioxide was added thereto, followed by further heating under reflux at a temperature in the range of 90 to 100°C for 2 hr, and thereby a green brown solution in which antimony trioxide was dissolved was obtained. Subsequently, the solution was cooled to a temperature of 15 to 20°C, followed by gradually and simultaneously adding under agitation 17.49 g of cesium hydroxide monohydrate dissolved in 100 ml of purified water and 20.88 g of ammonium acetate dissolved in 100 ml of purifiedwater, further followed by ripening at a temperature of 15 to 20°C for 1 hr, and thereby a brownish yellow slurry containing a cesium salt of a heteropoly acid precursor and an ammonium salt was obtained.

Subsequently, the slurry was dried by evaporation to dryness by use of hot water, followed by pulverizing with a mortar, further followed by classifying to 700 $\mu$m or less, and thereby pulverized matter was obtained. Acompositionof the pulverized matter was, in terms of charge ratio,

$$Mo_{10}V_{0.8}P_{1.15}Sb_{0.05}Cs_{0.5}(NH_4)_{1.3}.$$

Then, to the pulverized matter, 16.64 g of cupric acetate monohydrate was added in powder, followed by adding 100 g of an aqueous solution of 90% by mass of ethanol, further followed by kneading, still further followed by once more evaporating to dryness with hot water, followed by pulverizing with a mortar to 700 $\mu$m or less, further followed by subjecting to a primary calcining under air flow at 310°C for 5 hr, and thereby calcined granule (G) was obtained. A composition of the calcined granule (G) at this time was, in terms of charge ratio,

$$Mo_{10}V_{0.8}P_{1.15}Cu_{0.4}Sb_{0.05}Cs_{0.5}(NH_4)_{1.3}.$$

Subsequently, 366.4 g of the obtained calcined granule (G) was dispersed in 1360 ml of purified water, followed by agitating at 40°C for 1 hr. Then, the dispersion liquid was filtered, and filtered pale yellow water-insoluble matter (filtration residue) and a umber filtrate were separately evaporated to dryness with hot water, followed by pulverizing to 300 to 700 $\mu$m, further followed by classifying, and thereby granular catalyst (G) was obtained from the filtration residue, and solidified matter for comparison (G) was obtained from the filtrate.

At this time, a separation ratio between the filtration residue and the filtrate was 81.8% by mass of the filtration residue and 18.2% by mass of the filtrate.

Subsequently, 259.2 g of one that was obtained by pulverizing and classifying the obtained granular catalyst (G) to 300 $\mu$m or less and 38.4 g of a strength improver (ceramic fiber) were blended uniformly, followed by coating and molding on 318.2 g of spherical porous alumina carrier (particle diameter 3.5 mm) by use of an aqueous solution of 90% by mass of ethanol as a binder. Then the obtained molded body was divided equally into two, (i) one was calcined (secondary calcining) in a box type hot air calcining furnace under nitrogen flow (5 L/min) in the presence of ethanol (20 g/hr) as a reducing agent at 380°C for 10 hr, and thereby a targeted coated catalyst (G-1) was obtained, and (ii) the other was subjected to secondary calcining in a box type hot air calcining furnace under air flow at 380°C for 10 hr, and thereby a targeted coated catalyst (G-2) was obtained.

2) Catalytic oxidation of methacrolein

The obtained coated catalysts (G-1) and (G-2) each were used in a catalytic oxidation of methacrolein similarly to example 1, and reaction results were measured in the same way as example 1. The results are shown in Table 7. Furthermore, 4.0 g of the calcined granule (G) and the solidified matter for comparison (G) each and 8.0 g of the quartz sand of 300 to 700 $\mu$m were blended and used in the oxidation reaction under the same reaction conditions as the above. The results are shown together in Table 7.

[Table 7]

|  | Peak temperature (°C) | Conversion ratio (%) | Selectivity (%) | Yield (%) |
|---|---|---|---|---|
| Coated catalyst (G-1) | 321 | 76.6 | 86.7 | 66.4 |
| Coated catalyst (G-2) | 321 | 71.4 | 87.0 | 62.1 |
| Granular catalyst (G) | - | 68.5 | 89.6 | 61.1 |
| Solidified matter for comparison (G) | - | 42.1 | 85.1 | 35.9 |

(Comparative example 4)

1) Preparation of catalyst

In 2100 ml of purified water, 300 g of molybdenum trioxide, 15.16 g of vanadium pentoxide and 27.62 g of 85% by mass orthophosphoric acid were added, followed by heating under reflux at a temperature of 90 to 100°C for 5 hr, and thereby a umber clear solution was obtained. Then, 1.52 g of antimony trioxide was added thereto, followed by further heating under reflux at a temperature of 90 to 100°C for 2 hr, and thereby a green brown solution in which antimony trioxide was dissolved was obtained. Subsequently, the solution was cooled to a temperature of 15 to 20°C, followed by gradually and simultaneously adding under agitation 17.49 g of cesium hydroxide monohydrate dissolved in 100 ml of purified water and 20.88 g of ammonium acetate dissolved in 100 ml of purified water, further followed by ripening at a temperature of 15 to 20°C for 1 hr, and thereby an aeruginous slurry containing a cesium salt of a heteropoly acid precursor and an ammonium salt was obtained.
Subsequently, the slurry was dried by evaporation to dryness by use of hot water, followed by pulverizing with a mortar, further followed by classifying to 300 $\mu$m or less, and thereby pulverizedmatterwas obtained. Acompositionof the pulverized matter was, in terms of charge ratio,

$$Mo_{10}V_{0.8}P_{1.15}Cu_{0.4}Sb_{0.05}Cs_{0.5}.$$

Then, to the pulverized matter, 16.64 g of cupric acetate monohydrate was added in powder, and thereby granule for molded catalyst containing copper element was obtained. A composition thereof was, in terms of charge ratio,

$$Mo_{10}V_{0.8}P_{1.15}Cu_{0.4}Sb_{0.05}Cs_{0.5} (NH_4)_{1\cdot3}.$$

Subsequently, 361.0 g of the pulverized matter and 51.2 g of a strength improver (ceramic fiber) were blended uniformly, followed by coating and molding on 344.0 g of spherical porous alumina carrier (particle diameter 3.5 mm) by use of an aqueous solution of 90% by mass of ethanol as a binder. Then the obtained molded body was divided equally into two, (i) one was calcined (secondary calcining) in a box type hot air calcining furnace under nitrogen flow (5 L/min) in the presence of ethanol (20 g/hr) as a reducing agent at 380°C for 10 hr, and thereby a targeted coated catalyst (H-1) was obtained, and (ii) the other was subjected to secondary calcining in a box type hot air calcining furnace under air flow at 380°C for 10 hr, and thereby a targeted coated catalyst (H-2) was obtained.

2) Catalytic oxidation of methacrolein

The obtained coated catalysts (H-1) and (H-2) each were used in a catalytic oxidation of methacrolein similarly to example 1, and reaction results were measured in the same way as example 1. The results are shown in Table 8.

[Table 8]

|  | Peak temperature (°C) | Conversion ratio (%) | Selectivity (%) | Yield (%) |
|---|---|---|---|---|
| Coated catalyst (H-1) | 318 | 59.7 | 85.1 | 50.8 |
| Coated catalyst (H-2) | 317 | 56.9 | 83.5 | 47.5 |

[Example 5]

1) Preparation of catalyst

In 2450 ml of purified water, 350 g of molybdenum trioxide, 17.69 g of vanadium pentoxide and 32.23 g of 85% by mass orthophosphoric acid were added, followed by heating under reflux at a temperature of 90 to 100°C for 5 hr, and thereby a umbered clear solution was obtained. Subsequently, the solution was cooled to a temperature of 15 to 20°C, followed by gradually and simultaneously adding under agitation 20.41 g of cesium hydroxide monohydrate dissolved in 115 ml of purified water and 39.35 g of ammonium acetate dissolved in 175 ml of purified water, further followed by ripening at a temperature of 15 to 20°C for 1 hr, and thereby a yellowish-white slurry containing a cesium salt of a heteropoly acid precursor and an ammonium salt was obtained.

Then, 19.41 g of cupric acetate monohydrate dissolved in 240 ml of purified water was further added to the slurry, further followed by ripening at a temperature of 15 to 20°C for 15 min.

In the next place, the slurry was dried by evaporation to dryness by use of hot water, followed by pulverizing with a mortar, further followed by classifying to 700 $\mu$m or less, still further followed by subjecting to the primary calcining under air flow at 310°C for 5 hr, and thereby calcined granule was obtained. A composition of the calcined granule was, in terms of charge ratio,

$$Mo_{10}V_{0.8}P_{1.15}Cu_{0.4}Sb_{0.05}Cs_{0.5} (NH_4)_{2.1}.$$

Subsequently, 421.9 g of the obtained calcined granule was dispersed in 2220 ml of purified water, followed by agitating at 70°C for 3 hr. Then, the dispersion liquid was filtered, and filtered pale yellow water-insoluble matter (filtration residue) and a umber filtrate were separately evaporated to dryness with hot water, followed by pulverizing to 300 to 700 $\mu$m, further followed by classifying, and thereby granular catalyst (I) was obtained from the filtration residue, and solidified matter for comparison (I) was obtained from the filtrate.

At this time, a separation ratio of the filtration residue and the filtrate was 82.6% by mass for the filtration residue and 17.4% by mass for the filtrate.

Subsequently, 345.0 g of one obtained by pulverizing and classifying the obtained granular catalyst (I) to 300 $\mu$m or less and 48.8 g of a strength improver (ceramic fiber) were blended uniformly, followedbycoatingandmoldingon327.8gofspherical porous alumina carrier (particle diameter 3.5 mm) by use of an aqueous solution of 90% by mass of ethanol as a binder. Then the obtained molded body was divided equally into two, (i) one was pre-calcined under air flow at 273°C for 3 hr, followed by nitrogen calcining as the secondary calcining under nitrogen flow at 410°C for 7 hr, further followed by calcining under air flow at 370°C for 3 hr, and thereby a targeted coated catalyst (I-1) was obtained, and (ii) the other was subjected to secondary calcining under air flow at 383°C for 5 hr, and thereby a targeted coated catalyst (I-2) was obtained.

2) Catalytic oxidation of methacrolein

Obtained coated catalysts (I-1) and (I-2) were used in the catalytic oxidation of methacrolein similarly to example 1, and reaction results were measured similarly to example 1. Results thereof are shown in Table 9. Furthermore, 4.0 g of each of the granular catalyst (I) and the solidified matter for comparison (I) was mixed with 8.0 g of quartz sand of 300 to 700 $\mu$m, each of the mixture was used in the oxidation reaction under the same reaction conditions as that of example 1. Results are shown together in Table 9.

[Table 9]

| | Peak temperature (°C) | Conversion ratio (%) | Selectivity (%) | Yield (%) |
|---|---|---|---|---|
| Coated catalyst (I-1) | 322 | 83.9 | 86.8 | 72.8 |
| Coated catalyst (I-2) | 323 | 80.1 | 86.7 | 69.4 |
| Granular catalyst (I) | - | 70.0 | 87.7 | 61.4 |
| Solidified matter for comparison (I) | - | 12.2 | 73.7 | 9.0 |

ADVANTAGES OF THE INVENTION

A catalyst obtained by the preparation method according to the invention enables to producing methacrylic acid at high yield and high selectivity from methacrolein, isobutyl aldehyde or isobutyric acid. Furthermore, it can be used in a reaction under high load conditions and thus has a very large industrial value.

**Claims**

1. A method for preparing a coated catalyst for producing methacrylic acid by subjecting methacrolein, isobutyl aldehyde or isobutyric acid to gas phase catalytic oxidation, the method comprising:

   (a) a step of blending compounds each containing any one of Mo, V, P, Cu, CS or $NH_4$ and, as needed, a compound containing a metal element other than the above with water to prepare an aqueous solution or dispersion of the compounds (hereinafter referred to, both included, as a slurry);
   (b) a step of drying the slurry obtained in the step (a) to obtain a dried slurry;
   (c) a step of calcining the dried slurry obtained in the step (b) to obtain a calcined body;
   (d) a step of filtering a mixture obtained by blending the calcined body obtained in the step (c) with water to separate an aqueous solution and water-insoluble matter;
   (e) a step of drying the water-insoluble matter obtained in the step (d) to obtain a dried water-insoluble body; and
   (f) a step of coating a carrier with the dried water-insoluble body obtained in the step (e) using a binder to obtain a coated molded product.

2. The method according to claim 1, comprising: the steps (a) to (f) and

   (g) a step of calcining the coated molded product obtained in the step (f) under an inert gas atmosphere, under an air atmosphere or in the presence of a reducing agent.

3. The method according to claim 2, wherein the step (g) is a step of calcining the coated molded product obtained in the step (f) under an inert gas atmosphere.

4. The method according to any one of claims 1 to 3, wherein the compound containing Cu is cupper acetate or cupper oxide.

5. The method according to any one of claims 1 to 4, wherein the compound containing Cs is a weak acid salt of cesium or cesium hydroxide, a compound containing $NH_4$ is ammonium acetate or ammonium hydroxide.

6. The method according to any one of claims 1 to 5, wherein, the compound used as an optional component in the step (a) is a compound containing one or more kinds of elements selected from the group consisting of Sb, As, Ag, Mg, Zn, Al, B, Ge, Sn, Pb, Ti, Zr, Cr, Re, Bi, W, Fe, Co, Ni, Ce, Th, K and Rb.

7. The method according to any one of claims 1 to 6, wherein the slurry does not contain an arsenic compound.

8. The method according to any one of claims 1 to 7, wherein the binder is a binder containing ethanol.

**Patentansprüche**

1. Verfahren zur Herstellung eines beschichteten zur Herstellung von durch katalytische Gasphasenoxidation von Methacrolein, Isobutylaldehyd oder Isobuttersäure, umfassend:

   (a) einen Schritt der Vermischung von Verbindungen, enthaltend jeweils Mo, V, P, Cu, Cs oder $NH_4$ und, soweit erforderlich, eine Verbindung, enthaltend ein von den vorstehenden unterschiedliches Metall-Element, mit Wasser zur Herstellung einer wässrigen Lösung oder Suspension der Verbindungen (nachfolgend, beide einschließend, als Aufschlämmung bezeichnet);
   (b) einen Schritt der Trocknung der aus Schritt (a) erhaltenen Aufschlämmung zum Erhalt einer trockenen Aufschlämmung;
   (c) einen Schritt der Kalzinierung der aus Schritt (b) erhaltenen trockenen Aufschlämmung zum Erhalt eines kalzinierten Körpers;
   (d) einen Schritt der Filtrierung einer durch Mischen des aus Schritt (c) erhaltenen kalzinierten Körpers mit Wasser erhaltenen Mischung, zur Trennung einer wässrigen und wasserunlöslicher Anteile;

(e) einen Schritt der Trocknung der aus Schritt (d) erhaltenen wasserunlöslichen Anteile zum Erhalt eines getrockneten wasserunlöslichen Körpers; und

(f) einen Schritt der Beschichtung eines Trägers mit dem aus Schritt (e) erhaltenen getrockneten wasserunlöslichen Körper unter Verwendung eines Bindemittels zum Erhalt eines beschichteten geformten Produkts.

2. Verfahren nach Anspruch 1, umfassend:
die Schritte (a) bis (f) und

(g) einen Schritt der Kalzinierung des aus Schritt (f) erhaltenen beschichteten geformten Produkts unter inerter Gasatmosphäre, unter Luftatmosphäre oder in Gegenwart eines Reduktionsmittels.

3. Verfahren nach Anspruch 2, worin Schritt (g) ein Schritt der Kalzinierung des aus Schritt (f) erhaltenen beschichteten geformten Produkts unter inerter Gasatmosphäre ist.

4. Verfahren nach einem der ansprüche 1 bis 3, worin die Cu-haltige Verbindung Kupferacetat oder Kupferoxid ist.

5. Verfahren nach einem der ansprüche 1 bis 4, worin die Cs-haltige Verbindung ein schwach saures Salz von Cäsium oder Cäsiumhydroxid, eine $NH_4$-haltige Verbindung Ammoniumacetat oder Ammoniumhydroxid ist.

6. Verfahren nach einem der Ansprüche 1 bis 5, worin die in Schritt (a) optional eingesetzte Verbindung eine Verbindung ist, enthaltend eines oder mehrere der Elemente ausgewählt aus der Gruppe bestehend aus Sb, As, Ag, Mg, Zn, Al, B, Ge, Sn, Pb, Ti, Zr, Cr, Re, Bi, W, Fe, Co, Ni, Ce, Th, K und Rb.

7. Verfahren nach einem der Ansprüche 1 bis 6, worin die Aufschlämmung keine Arsenverbindung enthält.

8. Verfahren nach einem der Ansprüche 1 bis 7, worin das Bindemittel ein Ethanol-haltiges Bindemittel ist.

**Revendications**

1. Procédé de préparation d'un catalyseur revêtu pour la production de l'acide méthacrylique par l'oxydation catalytique en phase gazeuse de la méthacroléine, de l'aldéhyde d'isobutyle ou de l'acide isobutyrique, le procédé comprenant :

(a) une étape de mélange des composés contenant chacun l'un de Mo, V, P, Cu. CS ou $NH_4$ et, selon le besoin, un composé contenant un élément métallique autre que ceux mentionnés ci-dessus de l'eau pour préparer une solution ou une dispersion des composés (ci-aprés dénommé « la boue », incluant les deux) ;
(b) une étape de séchage de la boue obtenue à l'étape (a) pour obtenir une boue séchée :
(c) une étape de calcination de la boue séchée obtenue à l'étape (b) pour obtenir un corps calcine ;
(d) une étape de filtration une mélange obtenue par mélanger le corps calciné obtenu à l'étape (c) avec de l'eau pour séparer une solution aqueuse et des matières insolubles dans l'eau ;
(e) une étape de séchage les matières insolubles dans l'eau obtenues à (d) pour obtenir un corps séché insoluble dans l'eau ; et
(f) une étape de revêtement d'un corps porteur avec du corps séché insoluble dans l'eau obtenu à l'étape (e) en utilisant un liant pour obtenir une produis moulé revêtu,

2. Procédé selon la revendication 1 comprenant :
les étapes (a) à (f) et

(g) une étape de calcination du produit moulé revêtu obtenu à l'étape (f) sous atmosphère de gaz inerte, sous atmosphère de l'air ou en présence d'en agent réducteur.

3. Procédé selon la revendication 2, dans lequel l'étape (g) est une étape de calcination du produit moulé revêtu obtenu à l'étape (f) sous atmosphère de gaz inerte.

4. Procédé selon l'une des revendications 1 à 3, dans lequel le composé contenant du Cu est de l'acétate de cuivre ou de l'oxyde de cuivre.

5. Procédé selon l'une des revendications 1 à 4, dans lequel le composé contenant du Cs est un sel d'n acide faible

EP 1 629 889 B1

de césium ou de l'hydroxyde de césium, le composé contenant de NH$_4$ est de l'acétate d'ammonium ou de l'hydroxyde d'ammonium,

6.  Procédé selon l'une des revendications 1 à 5, dans lequel le composé utilisé comme composé optionnel à l'étape (a) est un composé contenant un ou plus éléments sélectionnés parmi le groupe consistant en Sb, As, Ag, Mg, Zn, Al, B, Ge, Sn, Pb, Tl, Zr, Cr, Re, Bi, W, Fe, Co, Ni, Ce, Th, K et Rb.

7.  Procédé selon l'une des revendications 1 à 6, dans lequel la boue ne comprend pas de composé arsénique,

8.  Procédé selon l'une des revendications 1 à 7, dans lequel le liant est un liant contenant de l'éthanol,

# EP 1 629 889 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 58011416 A
- JP 59024140 A
- JP 62014535 A
- JP 62030177 A
- JP 5031368 A
- US 5198579 A
- JP 8196908 A
- JP 11226411 A